# EUROPEAN PATENT SPECIFICATION

(11) **EP 1 956 914 B1**
(45) Date of publication and mention of the grant of the patent: **08.01.2014**
(21) Application number: 06838624.2
(22) Date of filing: 29.11.2006
(51) Int. Cl.: A01N 63/00, A61K 35/74, A61P 1/12, C12R 1/07, C12R 1/125

(54) **THE USE OF BACILLUS PB6 FOR THE PROPHYLAXIS OR TREATMENT OF GASTROINTESTINAL AND IMMUNO-RELATED DISEASES**
VERWENDUNG VON BACILLUS PB6 FÜR DIE PROPHYLAXE ODER BEHANDLUNG VON MAGEN-DARM-KRANKHEITEN UND IMMUNOLOGISCHEN KRANKHEITEN
UTILISATION DE BACILLUS PB6 POUR PREVENIR OU TRAITER DES MALADIES GASTRO-INTESTINALES OU DES MALADIES LIEES A L'IMMUNITE

(30) Priority: 29.11.2005 US 740518 P
(43) Date of publication of application: 20.08.2008
(73) Proprietor: KEMIN INDUSTRIES, INC., Des Moines, IA 50317 (US)
(72) Inventor: SAS, Benedikt, B-9190 Stekene (BE); VAN HEMEL, Johan, B-2600 Antwerpen (BE); VANDENKERCKHOVE, Jan, B-3271 Zichem (BE); PEYS, Eric, B-2490 Balen (BE); TAN, Hai Meng, Singapore S588132 (SG); SE, Chea-yun, Pontian Johor, 8200 (MY); RAMCHAND, Chaniyilparampu, Chennai 600 102 (IN); VARGHESE, Jerry, PO Kaffor, Kerala 680 702 (IN)
(74) Representative: Evans, Jacqueline Gail Victoria
(86) International application number: PCT/US2006/045755
(87) International publication number: WO 2007/064741

(56) References cited:
- WO-A1-2005/059112
- WO-A2-2004/050832
- JP-A- 2004 215 567
- JP-A- 2005 198 518
- US-B1- 6 461 607
- TEO A Y-L ET AL: "Inhibition of Clostridium perfringens by a novel strain of Bacillus subtilis isolated from the gastrointestinal tracts of healthy chickens", APPLIED AND ENVIRONMENTAL MICROBIOLOGY, AMERICAN SOCIETY FOR MICROBIOLOGY, US, vol. 71, no. 8, 1 August 2005 (2005-08-01) , pages 4185-4190, XP002375508, ISSN: 0099-2240, DOI: 10.1128/AEM.71.8.4185-4190.2005

### SEQUENCE LISTING

<110> Kemin Industries, Inc.
<120> KEM 121
<130> Kemin / P: KEM 121
<150> US 60/740,518
   <151> 2005-11-29
<160> 3
<170> PatentIn version 3.3
<210> 1
   <211> 1448
   <212> DNA
   <213> Bacillus subtilis
<400> 1
   aaaggttacc tcaccgactt cgggtgttac aaactctcgt ggtgtgacgg gcggtgtgta     60
   caaggcccgg gaacgtattc accgcggcat gctgatccgc gattactagc gattccagct    120
   tcacgcagtc gagttgcaga ctgcgatccg aactgagaac agatttgtgg gattggctta    180
   acctcgcggt ttcgctgccc tttgttctgt ccattgtagc acgtgtgtag cccaggtcat    240
   aaggggcatg atgatttgac gtcatcccca ccttcctccg gtttgtcacc ggcagtcacc    300
   ttagagtgcc caactgaatg ctggcaacta agatcaaggg ttgcgctcgt tgcgggactt    360
   aacccaacat ctcacgacac gagctgacga caaccatgca ccacctgtca ctctgccccc    420
   gaaggggacg tcctatctct aggattgtca gaggatgtca agacctggta aggttcttcg    480
   cgttgcttcg aattaaacca catgctccac cgcttgtgcg ggcccccgtc aattcctttg    540
   agtttcagtc ttgcgaccgt actccccagg cggagtgctt aatgcgttag ctgcagcact    600
   aaggggcgga aaccccctaa cacttagcac tcatcgttta cggcgtggac taccagggta    660
   tctaatcctg ttcgctcccc acgctttcgc tcctcagcgt cagttacaga ccagagagtc    720
   gccttcgcca ctggtgttcc tccacatctc tacgcatttc accgctacac gtggaattcc    780
   actctcctct tctgcactca agttccccag tttccaatga ccctccccgg ttgagccggg    840
   ggctttcaca tcagacttaa gaaaccgcct gcgagccctt tacgcccaat aattccggac    900
   aacgcttgcc acctacgtat taccgcggct gctggcacgt agttagccgt ggctttctgg    960
   ttaggtaccg tcaaggtgcc gccctatttg aacggcactt gttcttccct aacaacagag   1020
   ctttacgatc cgaaaacctt catcactcac gcggcgttgc tccgtcagac tttcgtccat   1080
   tgcggaagat tccctactgc tgcctcccgt aggagtctgg gccgtgtctc agtcccagtg   1140
   tggccgatca ccctctcagg tcggctacgc atcgtcgcct tggtgagccg tgacctcacc   1200
   aactagctaa tgcgccgcgg gtccatctgt aagtggtagc cgaagccacc ttttatgtct   1260
   gaaccatgcg gttcaaacaa ccatccggta ttagccccgg tttcccggag ttatcccagt   1320
   cttacaggca ggttacccac gtgttactca cccgtccgcc gctaacatca gggagcaagc   1380
   tcccatctgt ccgctcgact tgcatgtatt aggcacgccg ccagcgttcg tcctgagcca   1440
   tggatcaa                                                            1448
<210> 2
   <211> 1016
   <212> DNA
   <213> Bacillus subtilis
<400> 2
   gtcaggaaat gcgtacgtcc ttcctggact atgcaatgag cgttatcgta tcccgggcgc 60
   ttccggatgt gcgtgacggt ctgaagccgg ttcacagacg gattttgtac gcaatgaatg 120
   atttaggcat gaccagtgac aaaccatata aaaaatctgc ccgtatcgtc ggtgaagtta 180
   tcggtaagta ccacccgcac ggtgactcag cggtttacga atcaatggtc agaatggcgc 240
   aggattttaa ctaccgctac atgcttgttg acggacacgg caacttcggt tcggttgacg 300
   gcgactcagc ggccgcgatg cgttacacag aagcgagaat gtcaaaaatc gcaatggaaa 360
   ttctgcgtga cattacgaaa gacacgattg actatcaaga taactatgac ggttcagaaa 420
   gagagcctgc cgtcatgcct tcgagatttc cgaatctgct cgtaaacggg gctgccggta 480
   ttgcggtcgg aatggcgaca aacattcccc cgcatcagct tggagaagtc attgaaggcg 540
   tgcttgccgt aagtgagaat cctgagatta caaaccagga gctgatggag tacatcccgg 600
   gcccggattt tccgactgca ggtcagattt tgggccggag cggcatccgc aaggcgtatg 660
   aatccggacg gggatcaatc acaatccggg ctaaggctga aatcgaagag acttcatcgg 720
   gaaaagaaag aattattgtc acggaacttc cttatcaggt gaacaaagcg agattaattg 780
   aaaaaatcgc ggatcttgtc cgggacaaaa aaatcgaagg aattaccgat ctgcgagacg 840
   aatccgaccg taacggaatg agaatcgtca ttgagatccg ccgtgacgcc aatgctcacg 900
   tcattttgaa taacctgtac aaacaaacgg ccctgcagac gtctttcggg atcaacctgc 960
   tggcgctcgt tgacggacag ccgaaggtac taagcctgaa gcaatgcctg gagcat 1016
<210> 3
   <211> 801
   <212> DNA
   <213> Bacillus subtilis
<400> 3
   ataaaaaatc tgcccgtatc gtcggtgaag ttatcggtaa gtaccacccg cacggtgact 60
   cagcggttta cgaatcaatg gtcagaatgg cgcaggattt taactaccgc tacatgcttg 120
   ttgacggaca cggcaacttc ggttcggttg acggcgactc agcggccgcg atgcgttaca 180
   cagaagcgag aatgtcaaaa atcgcaatgg aaattctgcg tgacattacg aaagacacga 240
   ttgactatca agataactat gacggttcag aaagagagcc tgccgtcatg ccttcgagat   300
   ttccgaatct gctcgtaaac ggggctgccg gtattgcggt cggaatggcg acaaacattc   360
   ccccgcatca gcttggagaa gtcattgaag gcgtgcttgc cgtaagtgag aatcctgaga   420
   ttacaaacca ggagctgatg gagtacatcc cgggcccgga ttttccgact gcaggtcaga   480
   ttttgggccg gagcggcatc cgcaaggcgt atgaatccgg acggggatca atcacaatcc   540
   gggctaaggc tgaaatcgaa gagacttcat cgggaaaaga aagaattatt gtcacggaac   600
   ttccttatca ggtgaacaaa gcgagattaa ttgaaaaaat cgcggatctt gtccgggaca   660
   aaaaaatcga aggaattacc gatctgcgag acgaatccga ccgtaacgga atgagaatcg   720
   tcattgagat ccgccgkgac gccaatgctc acgtcatttt gaataacctg tacaamcaaa   780
   cggccctgca gaystctttc g                                             801

## Description

This application claims priority to United States Patent Application Serial No. 60/740,518, filed November 29, 2005.

### Background of the Invention

The invention relates generally to the administration of bacteria to treat gastrointestinal disease and, more specifically, to the administration of bacteria of a strain of *Bacillus* subtilis ATCC PTA-6737 to treat inflammatory bowel disease.

### Summary of the Invention

Prophylaxis of inflammatory bowel disease, may be provided by administering an effective amount of a *Bacillus* bacteria that produces lipopeptides. The Bacillus bacteria may be administered as a probiotic and may be combined with other probiotics, such as inulin.

Using biochemical methods, and specifically API 50 CBH/L, a preferred *Bacillus* was putatively identified as *Bacillus subtilis.* Using 16S rRNA, the preferred *Bacillus* was also putatively identified as *Bacillus subtilis.* Using *gyrA*, the preferred Bacillus was putatively identified as *Bacillus amyloliquefaciens.* Prior to the *gyrA* assay, the preferred *Bacillus* was deposited with the ATCC and identified as *Bacillus subtilis.*

The invention relates to the use of a *Bacillus* bacteria of the strain identified as ATCC strain PTA-6737 in the manufacture of a medicament for the prophylaxis or treatment of inflammatory bowel disease. The preferred *Bacillus* PB6 can be positioned for several unmet medical needs due to its versatile and unique characteristics.

The invention also relates to the use of an isolated bacteria strain having the 16S rRNA sequence of SEQ ID NO. 1 in the manufacture of a medicament for the prophylaxis or treatment of inflammatory bowel disease.

WO 2004/050832 discloses the use of Bacillus subtilis PB6 against Clostridium difficile or Clostridium perfringens.

### Brief Description of the Figures

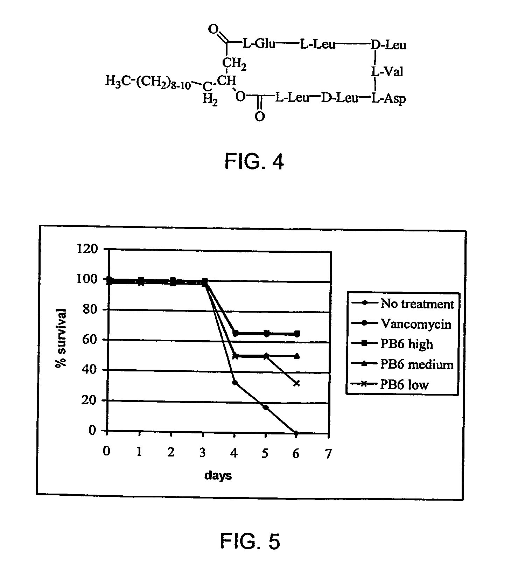

Fig. 4 is a drawing of the chemical structure of surfactin.
Fig. 6 is the 1466-bp 16S rRNA gene sequence (nucleotide position of 27-1492) of *Bacillus* PB6.
Fig. 7 is the 1023-bp partial gyrA sequence (nucleotide position of 43-1065) of *Bacillus* PB6.
Fig. 8 is the 801-bp consensus sequence obtained from partial *gyrA* sequencing of *Bacillus* PB6.
Fig. 9 is a gel of the detection of a PCR product (1650-bp) encoding for hemolysin BL; Lane 1, the GeneRuler™ mass ladders (3000, 2000, 1500, 1200-bp); Lane 2, *Bacillus* PB6; Lane 3, *Escherichia coli* ATCC 25922; Lane 4, *B. cereus* ATCC 49064; Lane 5, *B. cereus* ATCC 11778. No amplified band corresponding to a 1650-bp PCR product was detected in any of the lanes except for lanes 4 and 5.
Fig. 10 is a gel of the detection of a PCR product (1437-bp) encoding for non-hemolytic enterotoxin (Nhe). Lane 1, the GeneRuler™ mass ladders (3000, 2000, 1500, 1200-bp); Lane 2, *Bacillus* PB6; Lane 3, *Escherichia coli* ATCC 25922; Lane 4, *B. cereus* ATCC 49064; Lane 5, *B. cereus* ATCC 11778. No amplified band corresponding to a 1437-bp PCR product was detected in any of the lanes.
Fig. 11 is a gel of the detection of a PCR product (1400-bp) encoding for enterotoxin K (EntK); Lane 1, *Bacillus* PB6; Lane 3, *Escherichia coli* ATCC 25922; Lane 4, *B. cereus* ATCC 49064; Lane 5, *B. cereus* ATCC 11778; Lane 6, GeneRuler™ mass ladders (3000, 2000, 1500, 1200-bp); no amplified band corresponding to a 1400-bp PCR product was detected in any of the lanes.

### Detailed Description of Preferred Embodiments

PB6 is a proprietary bacterial strain that was isolated from nature and has not been genetically modified. Using a ribotyping technique this bacteria was identified as being a strain of *Bacillus subtilis.* DNA:DNA hybridization studies indicate that *Bacillus* PB6 strain may more likely be a *Bacillus amyloliquefaciens,* which will be further described below.

As used in this specification, the term prophylaxis means a medical or public health procedure whose purpose is to prevent rather than treat or cure a condition. As used in this specification, the term treatment means a medical or public health procedure whose purpose is to treat or cure a condition. As used in this specification, the term synergistic compound means a compound which enhances the prophylactic effect or treatment efficacy of a *Bacillus* bacterium administered for the prophylaxis or treatment of a disease or health condition. As used in this specification, lipopeptides are molecules that contain both lipids and proteins and include surface-active molecules containing several amino acids and one or more fatty acids. Surfactins, iturins, mycosubtilins, bafflomycins, bacillopeptins, fengycins, and plipastatins are examples of lipopeptides.

Filtrates from *Bacillus* PB6 were evaluated for the presence of hemolytic, non-hemolytic enterotoxins and enterotoxin K using commercially available immunological assays (TECRA and Oxoid). The same filtrates were further subjected to cytotoxicity tests on Vero and HEp-2 cell lines. Finally, PCR-based methods were used to confirm for the presence of genes with possible enterotoxigenic capacity in *Bacillus* PB6. There was no immuno-cross reactivity observed with Hbl or Nhe enterotoxins and antibodies in the two commercial immunoassays. No cytotoxicity was also observed with the Vero and HEp-2 cell assays. *Bacillus* PB6 strain does not produce the hemolytic, non-hemolytic enterotoxins and enterotoxin K under the same conditions that allowed detection for a known toxigenic strain of *B. cereus.*

The *in vivo* toxicity of *Bacillus* PB6 has also been tested. Therefore a spray-dried product containing 10¹⁰ cfu/g of *Bacillus* PB6 was made from the fermentation product.

### Toxicity Study of Spray-dried B. amyloliquefaciens in Wistar Rats

A first study was designed and conducted to determine the acute oral toxicity of the spray-dried *Bacillus* PB6 product (10¹⁰ cfu/g) in Wistar rats. A total of 5 male and 5 female animals were administered an oral dose of 5000 mg/kg (as a suspension in double distilled water and using a dose volume of 10 ml/kg). The control group consisted of 5 male and 5 female animals that received double distilled water at a dose of 10 ml/kg. No mortality was observed in the 5000 mg/kg treated animals. 50 % of the animals receiving the *Bacillus* PB6 product were more active in comparison with the control group. None of the animals treated with *Bacillus* PB6 suffered from diarrhea. After necroscopy, no pathological changes were seen in any organ in both groups. Thus the maximum non-lethal dose (LD₀) and LD₅₀ of the orally administered *Bacillus* PB6 product in Wistar rats was found to be greater than 5000 mg/kg.

Conclusion: Maximum non-lethal dose (LD₀) and LD₅₀ of *B.* PB6 dry (10¹⁰ cfu/g) in Wistar rat by oral route was found to be greater than 5000 mg/kg.

### Repeated Dose 28-day Oral Toxicity of B. PB6 in Wistar Rats

This study was designed and conducted to determine repeated dose (28 days) oral toxicity of *B.* PB6 (10¹⁰ cfu/g) in Wistar rats. In each group 6 male and 6 female animals were administered oral doses of 250, 500 or 1000 mg/kg for 28 days. The control vehicle group also consisted of 6 male and 6 female animals, which received doubled distilled water at a dose of 10 ml/kg for 28 days. These groups were sacrificed on day 29.

The study also consisted of two reversible groups for control vehicle and high dose which each had 6 male and 6 female animals. The high dose group received medication up to day 28 and was without treatment from day 29 to 42 and sacrificed on day 43. The control vehicle group received distilled water at a dose of 10ml/kg up to day 28 and was without treatment from day 29 to day 42 and sacrificed on day 43.

Conclusion: No Observed Adverse Event Level (NOAEL) for *B.* PB6 (10¹⁰ cfu/g) in 28 days toxicity trials in rats is found to be greater than 1000 mg/kg.

### Local Irritancy Study (Dermal and Eye) of B. PB6 in New-Zealand White Rabbit

A total of 3 New-Zealand White Rabbits (either sex) - Same rabbits were used for dermal and eye irritation in this study. A minimum gap of 5 days was kept between both studies. 1 g of *B*. PB6 (10¹⁰ cfu/g) was applied as a paste to the skin for dermal irritation and 100µl of 10% *B.* PB6 suspension instilled into left eye for eye irritation.

Test Item PB6 paste was applied to skin at dorso-lateral area after removal of hairs in 3 rabbits. Suspension of 10% PB6 was instilled in the eye of 3 rabbits. The test item was removed from skin of the animals 4 hours post-application. After instillation of test item in left eye, the eyelids were held together for 2-3 seconds.

The rabbits were observed and scored for dermal irritancy at 1, 24, 48 and 72 hours after removal of test item PB6. For eye irritancy scoring was carried out at the same time points post-instillation of PB6 suspension.

Conclusions: Dermal: No irritation was observed, when 1g of *B*. PB6 (10¹⁰ cfu/g) was applied as a paste.

Eye : No irritation was observed, when 100µl of 10% suspension of *B*. PB6 (10¹⁰ cfu/g) was instilled.

### Erythrocyte Micronucleus Assay of B. amyloliquefaciens PB6 in Mice

This study was designed and conducted to detect the damage induced by the test substance to the chromosomes or the mitotic apparatus of Swiss albino mice. A total of 5 male and 5 female animals were administered oral dose of 2500 and 5000mg/kg, the control vehicle group consisted of 5 male and 5 female mice which received double distilled water at a dose of 10ml/kg orally. The positive control group (5 male + 5 female) received cyclophosphamide orally at a dose of 40 mg/kg.

The animals were sacrificed by excess of CO₂ at respective time points (control group, 2500 mg/kg PB6 cyclophosphamide at 24h and 5000 mg/kg PB6 at both 24 and 48 h) and both femora were removed and bone marrow smears made on slides, stained with Giemsa and May-Greunwald stain, viewed under microscope for the incidence of micronucleus by counting 2000 immature erythrocytes.

The percentage of immature among total (immature + mature) erythrocytes is also determined for each animal by counting at least 200 erythrocytes.

Conclusion: *B*. PB6 (10¹⁰ cfu/g) at a dose of 2500 & 5000 mg/kg did not significantly induce micro nucleated polychromatic erythrocytes in mice.

### Evaluation of the Efficacious Components of the Orally Administered Bacillus PB6

The new dietary concept ofprobiotics (such as inulin) is gaining popularity among nutritionists, physicians, food manufactures and consumers. The most widely accepted definition of a probiotic food is: "A probiotic is a selectively fermented ingredient that allows specific changes, both in the composition and activity of the gastrointestinal microflora that confers benefits upon the host well-being and health." To be classified as a probiotic ingredient in food products, that ingredient must 1) resist digestion (hydrolysis) by alimentary enzymes in the stomach and small intestine; 2) enter the colon chemically intact and subsequently undergo partial or complete fermentation; and 3) stimulate active growth and/or activity of health-stimulating intestinal bacteria. Health benefits of probiotic-stimulated bacteria growth are wide-ranging and include such benefits as positive effects on colon cancer and pathogens, decreased triacylglycerols, increased absorption of Ca and Mg, and increased stool frequency and fecal weight. Inulin is a polydisperse β(2→ 1) fructan with chain lengths ranging from 2 to 60 units. The unique linkage between the fructose molecules of inulin and oligofructose distinguish them from typical carbohydrates. They resist digestion by human alimentary enzymes and absorption in the small intestine but are hydrolyzed and fermented by colonic microflora and therefore classified as a probiotic dietary fiber.^{42,43,44,45,46}

The fact that probiotics stimulate the active growth and/or activity of health stimulating intestinal bacteria, make them very interesting products to be administered together with probiotics such as *Bacillus* PB6.

In addition, *Bacillus amyloliquefaciens* PB6 does not inhibit the growth of "healthy" bacteria from the gut flora such as *LactoBacillus* spp. and *Bifidobacterium* spp.

### Materials and Methods

### Identification of isolated PB6 bacteria

The PB6 *Bacillus* strain isolated is a Gram-positive rod and possesses catalase. Malachite Green staining confirmed that this microorganism possesses endospores and is a spore-former. This microorganism is also a "swarmer" as upon extended incubation period, it tends to spread over the entire agar surface. In terms of relatedness to known *Bacillus* spp., the *Bacillus* strain isolated has 92.0 % ID. Based on the API biochemical profiles, the PB6 *Bacillus* strain was putatively identified as *B. subtilis.* The PB6 *Bacillus* strain was therefore deposited as ATCC - PTA 6737 and named there *Bacillus subtilis* (United States Patent Application Serial No. 10/306,365, filed November 27, 2002).

### 16 rRNA gene sequencing

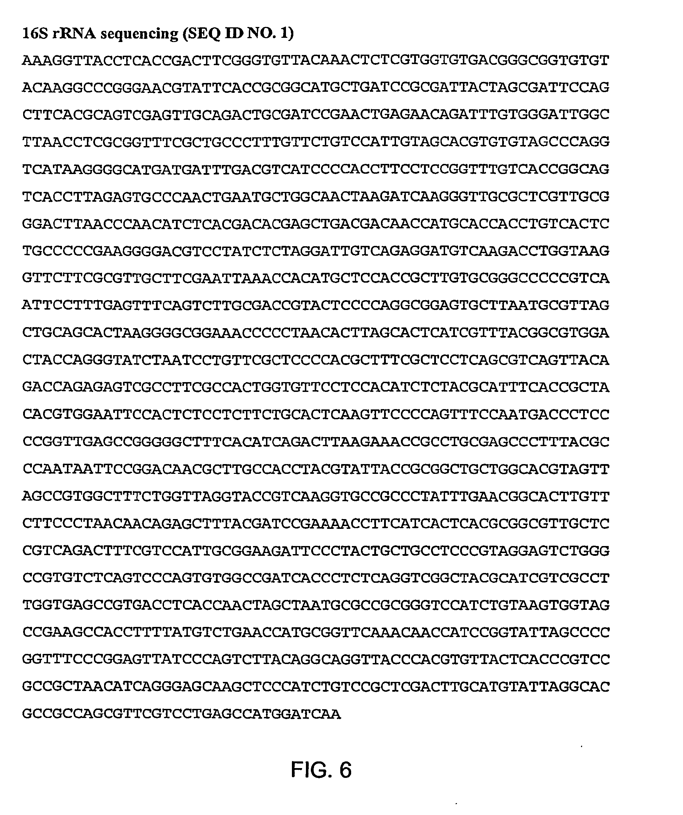

The nearly complete 16 rRNA sequence of *Bacillus* PB6 was completed and aligned (SEQ ID NO.1, Fig. 6).

### GyrA sequencing

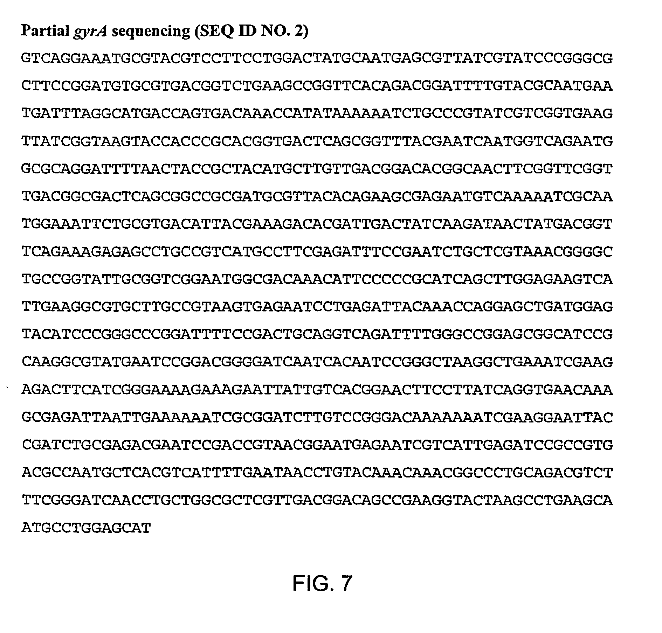

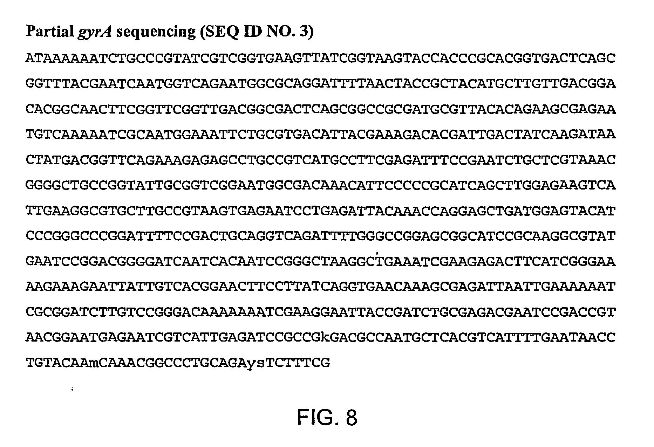

Partial gyrA sequences of PB6 obtained by two different groups are shown in Fig. 7 (SEQ ID No. 2) and Fig. 8 (SEQ ID NO. 3).

### DNA:DNA hybridizations

Hybridizations were performed under stringent conditions (at 40 °C) according to a modification of the method described by Ezaki *et al*.⁴⁷ The DNA homology percentages are the mean of minimum 4 hybridizations. The value given between brackets is the difference between the reciprocal values. With this technique, the average standard deviation is 14 units.⁴⁸ The results are presented in Table 7.

**Table 7 - %DNA homology**

| | % DNA homology | | |
|---|---|---|---|
| *Bacillus* PB6 | 100 | | |
| *B. amyloliquefaciens* LMG 9814^{T} | 75 (13) | 100 | |
| *B. velezensis* LMG 22478^{T} | 90 (6) | 80 (8) | 100 |

A DNA homology above 70%, the generally accepted limit for species delineation,⁴⁹ is found between *Bacillus* PB6, LMG 9814^{T} and LMG 22478^{T}.

From these results, it appears that *B. velezensis* and *B. amyloliquefaciens* belong to the same genospecies and are therefore subjective synonyms such that in applying nomenclatural rule 42 the oldest legitimate name should be retained, i.e. *B. amyloliquefaciens;* and that *Bacillus* PB6 (ATCC -PTA 6737) may more properly be categorized to the species *B. amyloliquefaciens.*

### Determination of enterotoxin production by Bacillus PB6

Bacterial strains and culture conditions. *Bacillus* PB6 was grown in 100-ml volume of Tryptic Soy Broth (Becton Dickenson and Company, Cockeysville, MD) supplemented with 0.6% yeast extract (Oxoid Limited, England) (TSBYE) and incubated at 37°C in a shaker incubator set at 100 rpm. One toxin-producing strain of *B. cereus* ATCC 11778 was also grown in TSBYE at 37°C in a shaker incubator. Similarly, non toxin-producing strains of *B. cereus* ATCC 49064 and *Escherichia coli* ATCC 25922 were also grown in TSBYE at 37°C under aerobic conditions. All bacterial strains used in this study were transferred weekly to fresh TSBYE and then kept in a 4°C refrigerator as working culture. Freshly grown strains were re-suspended in 40% glycerol and kept at-80°C freezer for long-term storage.

Enterotoxin production. A 1-ml volume of overnight test culture was inoculated into 50 ml of Brain Heart Infusion (BHI) supplemented with 1% glucose (BHIG) and incubated in a shaking incubator (100 rpm) for 6 h at 32°C³¹. Bacterial cells were precipitated by centrifugation at 5000 X *g* for 10 min and the supernatant was collected for cytotoxicity studies of Vero cells³¹. The proteins in the supernatant were then concentrated ten-fold using up to 80% saturated ammonium sulfate solution (561 g per liter)³³. After centrifugation at 10000 X g for 20 min, the supernatant was decanted and protein-pellet was re-suspended in 2.5 ml of phosphate buffer (20 mM; pH 6.8). Residual salts of ammonium sulfate were removed by dialysis against the same buffer at 4°C for 6 h. The final volume of the dialyzed protein solution was adjusted to one-tenth of the original volume (5 ml) using the phosphate buffer (20 mM; pH 6.8)³¹.

Emetic toxin production. A 1-ml volume of overnight test culture was inoculated into 50 ml of Brain Heart Infusion (BHI) supplemented with 1% glucose (BHIG) and incubated for 6 h at 32°C in a shaker incubator set at 250 rpm. Bacterial cells were precipitated by centrifugation at 2000 X *g* for 10 min at 4°C³¹. The supernatant was collected and then autoclaved at 121°C for 15 min to remove heat-labile enterotoxins²⁰. The heat-treated filtrate with possibly the heat-stable emetic toxin was collected for the vacuolation assay on HEp-2 cells²⁰.

Preparation of Vero and HEp-2 cells. African green monkey kidney cells (Vero) or HEp-2 (human carcinoma of the larynx) were maintained as monolayer cultures in 30 ml of Medium 199 with Earle's modified salts (MEM) containing 2 mM L-glutamine, 10 mM sodium bicarbonate, 1% non-essential amino acids, 100 UI/ml penicillin, 0.1 mg/ml streptomycin, and 3 ml fetal calf serum (10%). The leucine-free medium (MEM) was prepared on the basis of minimum essential medium (Gibco), which also contained 1.8 mM CaCl₂, 0.4 mM MgCl₂, 5.0 mM KCl, 0.12 M NaCl, 3.2 mM NaH₂PO₄, and 20 mM Hepes (pH 7.7). The Vero or HEp-2 cells were incubated in MEM at 5% CO₂ at 37°C. Confluent monolayer cultures of Vero or HEp-2 cells were sub-cultured by discarding the media before washing with 5 ml of PBS (pH 7.7). Vero or HEp-2 cells were then detached from the culture flask by the addition of 2 ml of trypsin solution (0.25% trypsin; 0.025% EDTA). The levels of loosen Vero or HEp-2 cells were determined microscopically before the addition of MEM medium (8 ml) to prevent further effect of trypsin. A 5-ml aliquot of freshly trypsinised Vero or HEp-2 cells were then transferred to new culture flask containing 15 ml of MEM for incubation at 37°C under 5% CO₂.

TECRA^{®} *Bacillus* Diarrheal Enterotoxin (BDE) Visual Immunoassay. All components of the test kit (TECRA International Pte Ltd, Chatswood, NSW, Australia) were kept at 20 - 25°C prior to testing of samples for BDE. As stated by the manufacturer, microtiter wells containing high affinity antibodies specific for BDE were pre-soaked with wash solution, provided in the kit and allowed to stand for 10 min at 20-25°C. The wells were emptied before aliquots containing 200-µl volume of test samples and controls (positive and negative) were transferred into individual wells. The wells were incubated at 37°C for 2 h. The wells were washed 4 times before an aliquot of 200 µl of conjugate was added to each well and incubated at 25°C for 1 h. Each well was washed 5 times before 200 µl of substrate was added to each well and incubated at 25°C for 30 min. After 30 min, the colorimetric development for each well was compared against the TECRA Color Card provided in the test kit.

Oxoid *Bacillus cereus* Enterotoxin Reversed Passive Latex Agglutination (BCET-RPLA). The test was developed for the detection of diarrheal enterotoxin of *Bacillus cereus* by reversed passive agglutination (RPLA) (Unipath, Basingstoke, UK). Polystyrene latex particles are sensitized with purified antiserum from rabbits immunized with diarrheal enterotoxin from *B. cereus.* The kit also provided the both positive (enterotoxin) and negative (latex particles without specific *B. cereus* anti-enterotoxin) controls. Aliquots containing 25 µl of diluent and test or control (positive and negative) samples were dispensed successively into 2 different sets of V-well microtiter plates. Solutions containing 25 µl of sensitized latex and latex control were then dispensed into the first and second set of V-well microtiter plates, respectively. Each well was examined for agglutination after 20 - 24 h of incubation at 25°C.

Measurement of cytotoxicity. Freshly trypsinised Vero cells were re-suspended in 30 ml of leucine-free medium (MEM). One milliliter of the Vero cell suspension was transferred to each of the 24 wells (~5 X 10⁴ cells per well). Cells were washed once with 1 ml of leucine-free MEM and incubated for 2 h at 37°C under 5% CO₂. After 2 h, the growth medium was removed and each well was washed once with 1 ml of leucine-free MEM. A 1-ml volume of preheated (37°C) leucine-free MEM was added to each well, followed by 50 µl of supernatant or filtrate of *Bacillus amyloliquefaciens* PB6, *B. cereus* ATCC 11778, *B. cereus* ATCC 49064 and *E. coli* ATCC 25922 immediately thereafter. The inoculated wells were incubated at 37°C with 5% CO₂ for 2 h. After 2 h of incubation, supernatant or filtrate-treated Vero cells were washed once with 1 ml of preheated (37°C) leucine-free MEM. A volume of 300 µl of solution containing radioactive-labeled isotope (16 µl ¹⁴C-leucine in 8 ml of leucine-free MEM) (Perkin Elmer Asia, Singapore) was added to each of the well. ¹⁴C-leucine labeled Vero cells were incubated at 37°C without CO₂ for 1 h and thereafter the growth medium was discarded. Subsequently, 1-ml aliquots of solution containing 5% trichloroacetic acid were added to each well containing ¹⁴C-leucine labeled Vero cells before incubating at 25°C for 10 min. After 10 min of incubation, the contents in each well were washed twice with 1 ml of 5% trichloroacetic acid-solution. A 300-µl volume of 0.1 M KOH was then added to each well and incubated at 25°C for another 10 min. After 10 min of incubation, 2-ml volume of scintillation liquid was added to each well. Finally, all contents from each well were transferred into scintillation tubes. All scintillation tubes were agitated for 1 min before performing radioactivity counts. The percent inhibition of ¹⁴C leucine uptake by Vero cells is calculated using the following formula.
Percent inhibition of ¹⁴C leucine uptake = [(cpm for Vero cells without toxin added - cpm for test simple)] / [cpm for Vero cells without toxin added] X 100%. The cpm used for calculating percent inhibition of ¹⁴C leucine uptake by Vero cells has to be subtracted by the value for background counts (~ 30 - 60 cpm). No toxin is present if the inhibition of ¹⁴C leucine uptake is less than 20% after the ten-fold concentration. Each assay was conducted in duplicates. The results are presented in Tables 8 and 9.

**Table 8: Effect of bacterial filtrate on Vero and HEp-2 Cell Lines**

| Bacteria | Vero cells | | HEp-2 cells | |
|---|---|---|---|---|
| | Cytotoxic effect | | Vacuole response | |
| | 1 h | 24 h | 6 h | 24 h |
| *Bacillus amyloliquefaciens* PB6 | -^{a} | - | - | - |
| *Escherichia coli* ATCC 25922 | - | - | - | - |
| *Bacillus cereus* ATCC 11778 | - | - | - | - |
| *Bacillus cereus* ATCC 49064 | ++^{b} | ++++ | - | +++ |

| | | | | |
|---|---|---|---|---|
| ^{a} "-" indicates no cytotoxic effect or presence of vacuoles observed. ^{b} "+" indicates destruction of Vero cell or vacuole production in HEp-2 cells have occurred. | | | | |

**Table 9: % inhibition of ¹⁴C-leucine isotope uptake**

| Concentrated filtrates of bacteria | % inhibition^{a} |
|---|---|
| *Bacillus amyloliquefaciens* PB6^{b} | 1 |
| *Escherichia coli* ATCC 25922^{c} | 1 |
| *Bacillus cereus* ATCC 11778^{d} | 17 |
| *Bacillus cereus* ATCC 49064^{e} | 100 |

| | |
|---|---|
| ^{a}Toxin from the tested strain is considered negative if the inhibition of ¹⁴C leucine uptake is less than 20% after ten-fold concentration. (SCAN report). ^{b}Test microorganisms, ^{c}negative and ^{e}positive controls and ^{d}non-toxin producing strain. | |

HEp-2 Cells Vacuolation Assay. A 25-µl volume containing filtrates from test and control cultures were serially diluted (2-fold) in 0.15 M NaCl solution and dispensed across wells of a 96-well tissue culture plate (Gibco Ltd, Uxbridge, UK). Volumes containing 100 µl of freshly trypsinized HEp-2 cells were added to each well and incubated for 24 h at 37°C. All vacuolation assays were performed in duplicates. Microscopic examination for the presence of vacuole formation in HEp-2 cells was conducted at the 6^{th} and 24^{th} hour-intervals.

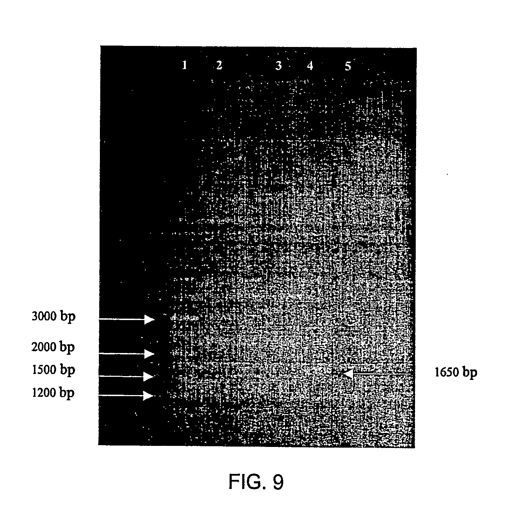

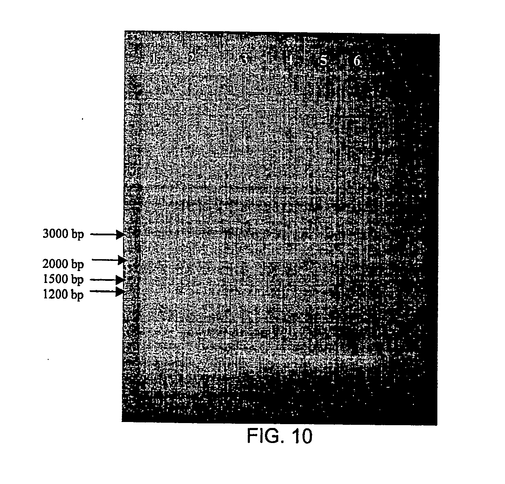

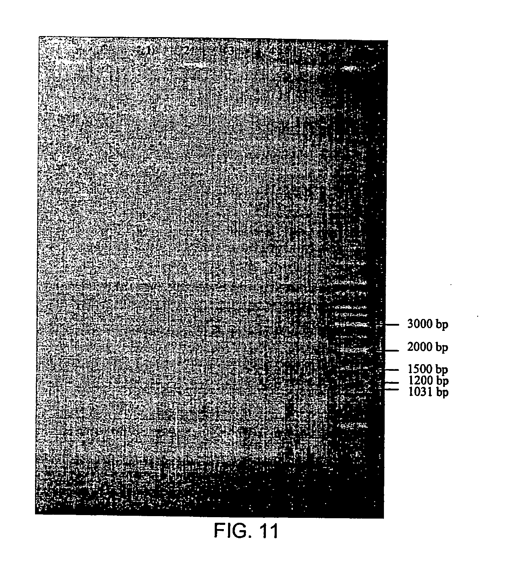

PCR-based methods. PCR primers were developed for the non-haemolytic enterotoxin (*NheB-nheC*)^{25,26}, hemolysin BL (*HblD-hblA*)^{10,11}, and enterotoxin K (*EntK*)^{27,28}. The sequences of the *NheB-nheC* primers were 5' CGGTTCATC-TGTTGCGACAGC 3' and 3'GTCCTCGTGTTCGTCTTC- AGC 5'. The primer sequences for *HblD-hblA* were 5' CGCT- CAAGAACAAAAAGTAGG 3'and 3' TCCCTAATGT- CTAAATGTTCCTC 5'. The forward and reverse primers for *EntK* were 5'GAATTACGTTGGCGAATC3' and 3' CGGG- CGGATGGGA 5', respectively. Both sample PCR and positive control tubes containing DNA of *Bacillus amyloliquefaciens* PB6 and toxigenic strains of *B. cereus* ATCC 11778 and *B. cereus* ATCC 49064 were placed into a Perkin-Elmer thermal cycler (GeneAmp PCR System 9600, Perkin-Elmer Corp., Norwalk, CT) with initial set point temperature of 90°C. The conditions for thermal cycling were 1 cycle at 94°C for 2 min followed by a 38-cycle temperature cycling routine of 94°C for 15 s, and 70°C for 3 min. Following amplification, a final extension at 72°C for 7 min was performed. The PCR amplification step required approximately 3 h to complete. Aliquots (15µl) of each reaction product and 15µl DNA standard ladder (500, 1031, 2000 and 3000 base-pairs) were then electrophoresed at 180 volts for 1 h through a 2% agarose gel (Life Technologies Inc., Gaithersburg, MD) containing 0.1 µg/mlethidiurn bromide (Sigma Chemical Co., St. Louis, MO). After electrophoresis, all gels were viewed using a UV-transilluminator (wavelength - 302 nm) (UVP Model White /2 UV; UVP Inc., Upland, CA) and then photographed using a Polaroid MP-4 Camera (Polaroid Corp., Cambridge, MA)(See Fig. 9, Fig. 10 and Fig. 11).

### EXAMPLE EFFICACY OF BACILLUS PB6 AGAINST IBD

It is known that surfactin inhibits the activity of cytosolic PLA₂, an enzyme centrally involved in many inflammatory processes.⁵⁸ The inhibition of inflammatory processes makes surfactin producing probiotics very interesting for the treatment of inflammatory diseases, such as Inflammatory Bowel Disease (IBD).

Inflammatory bowel disease refers to two chronic diseases that cause inflammation of the intestines: ulcerative colitis (UC) and Crohn's disease (CD). Although the diseases have some features in common, there are some important differences.

CD is a chronic inflammation of the intestinal wall, typically affecting the full thickness of the intestinal wall. Most commonly, it occurs in the lowest portion of the small intestine (ileum) and the large intestine, but it can occur in any part of the digestive tract from the mouth to the anus and the skin around the anus.

In recent decades, CD has become more common both in western and developing countries. It occurs roughly equally in both sexes, and is more common among Jewish people. Most cases begin before the age of 30; the majority starts between the ages of 14 and 24.

In each person, the disease affects specific areas of the intestine, sometimes with normal areas (skip areas) sandwiched between the affected zones. In about 35% of CD sufferers, only the ileum is affected. In about 20%, only the large intestine is affected. In about 45% of patients, both the ileum and the large intestine are affected.

The causes of CD are unknown. Research has focused on three main possibilities: a dysfunction of the immune system, infection, and diet.

The most common early symptoms of CD are chronic diarrhea, abdominal pain, fever, loss of appetite, and weight loss. Symptoms differ among CD patients, but there are four common patterns:
- inflammation with pain and tenderness in the right lower part of the abdomen;
- recurring acute intestinal obstructions that cause severe painful spasms of the intestinal wall, swelling of the abdomen, constipation, and vomiting;
- inflammation and chronic partial intestinal obstruction causing malnutrition and chronic debility;
- abnormal fistulas and abscesses that often cause fever, painful masses in the abdomen, and severe weight loss.

UC is a chronic disease in which the large intestine becomes inflamed and ulcerated, leading to episodes of bloody diarrhea, abdominal cramps, and fever. The disease can start at any age, but usually begins between the ages of 15 and 30.

Unlike CD, UC does not usually affect the full thickness of the intestine, and does not affect the small intestine. The disease usually begins in the rectum or the sigmoid colon, and eventually spreads partially or completely through the large intestine. In some patients, most of the large intestine is affected early on.

About 10% of patients who appear to have UC only suffer a single attack. However, a proportion of such patients may actually be suffering from an undetected infection, rather than true UC. For most patients, UC is a chronic disease that waxes and wanes over time. The causes ofUC remain unknown. Heredity and over-active immune responses in the intestine are thought to be contributing factors.

### Determination of the in vivo efficacy of orally administered Bacillus PB6 in the treatment of TNBS-induced colitis in rats (a model for colitis in humans).

In this experiment, the efficacy of PB6 was studied against colitis in rats induced by the rectal administration of 2,4,5-trinitrobenzene sulfonic acid (TNBS).

### Study design

Two consecutive trials were done. Male Wistar rats were obtained from the National Centre for Laboratory Animal Sciences (Hyderabad, India) (trial 1) or from the Department of Animal Medicine, TANUVAS (Chennai, India) (trial 2). At the beginning of the treatment period, the animals were 10 to 12 weeks old. Upon their arrival at the test facility, the animals were given a complete clinical examination under the supervision of a veterinarian to ensure that they were in good condition. The animals were acclimatised to the study conditions for a period of at least 7 days. Per trial, animals were randomised and allocated to one of the study groups. The animals were housed per study group in polycarbonate cages (421x290x190 mm, LxWxH). The animal room and test room conditions were set as follows: temperature: 22 ± 3°C, relative humidity: 50 ± 20%, light/dark cycle: 12hr/12hr (light 07.00-19.00) and ventilation: approximately 7 cycles/hour of filtered, non-recycled air. All animals had free access (except for the overnight fasting prior to TNBS administration) to rat pellet feed from the National Institute of Nutrition (NIN, Hyderabad, India) and Aquaguard purified water *ad libitum.* In both trials the day of induction of colitis was set as day 1. Colitis was induced, after an overnight fast, using a single intrarectal administration of TNBS at 100 mg/Kg body weight, 8 cm proximal to the anus. The colitis-negative control groups were given saline intrarectally (0.5 ml per animal once) on day 1. Colitis-negative and colitis-positive control groups were given distilled water orally at 10 ml/kg, 3 times daily with 4 h inter dosing, starting on day 1 and up to and including day 7. In the first trial, groups with TNBS-induced colitis were treated with PB6 (1.5 10⁸ CFU/Kg or 1.5 10⁹ CFU/Kg respectively), 3 times daily with 4 h interdosing, starting on day 1 and up to and including day 7; with mesalazine (250 mg/Kg/day), starting on day 1 and up to and including day 7; or with infliximab (3 mg/Kg) as a single dose on day 1. The second trial partially repeated the first one concerning the PB6 (1.5 10⁸ CFU/Kg), mesalazine and infliximab treatments and included an additional treatment with *S. boulardii* (1.5 10⁸ CFU/Kg), 3 times daily with 4 h interdosing, starting on day 1 and up to and including day 7. The first or only (in case of infliximab) dose of treatment was given within 2 (distilled water, PB6, *S. boulardii* and mesalazine) or 3 (infliximab) hours after administration of TNBS. Except for infliximab, which was injected intravenously, all treatments were administered by gavage. Twice daily observations were made for clinical signs and mortality. Body weights of animals were recorded on days 1, 4 and 7. On day 8 animals were sacrificed and a 5 cm long segment of the colon (from 10 to 5 cm proximal to the anus) was excised. These segments were opened longitudinally. Contents were removed by washing with saline and gross morphology was scored using the following scale: 0 - no ulcers or inflammation, 1 - no ulcers only local hyperaemia, 2 - ulceration without hyperaemia, 3 - ulceration and inflammation at one site only, 4 - two or more sites of ulceration and inflammation, and 5 - ulceration extending more than 2 cm. The weight of each 5 cm colonic segment was also recorded to assess inflammatory induced edema.

### Test preparations

TNBS 5% (w/v) in water (Sigma-aldrich, St Louis, USA) was diluted to a 2.5% solution with ethanol 50%. Dose volume was 4 ml/Kg body weight. PB6 Dry (Kemin Health, Des Moines, USA), a *Bacillus* 'PB6' fermentation broth dried on a malto- and cyclodextrin carrier, was suspended in distilled water to concentrations of 1.5 10⁷ and 1.5 10⁸ CFU/ml. Dose volume was 10 ml/Kg body weight. *Saccharomyces boulardii* (Enterol^{®}, Biodiphar, Brussel, Belgium) was suspended in distilled water to a concentration of 1.5 10⁷ CFU/ml. Dose volume was 10 ml/Kg body weight. Mesalazine (Mesacol^{®}, Sun Pharmaceutical Ind. Ltd, Mumbai, India) tablets were powdered using pestle and mortar and a solution in distilled water was prepared containing 25 mg 5-aminosalicylic acid per ml. Dose volume was 10 ml/Kg body weight. Remicade^{®} (Infliximab) (Centocor B.V., Leiden, The Netherlands) was first reconstituted with 10 ml water for injection and was further diluted to 2 mg/ml concentration using saline. Dose volume employed was 1.5 ml/Kg body weight. All body weight dependant doses were administered on the basis of the last individual body weight taken. Fresh preparations were made prior to each administration. The preparations were stirred vigorously before each dosing.

### Results and discussion

In the first trial one of the rats treated with Infliximab died on day 2. This animal had been given a second injection on day 1 because at the first one drug solution oozed out. Some animals showed mild signs of diarrhea after induction of colitis with TNBS. The number of observations of diarrhea recorded (and the number of animals affected) in the different treatment groups from day 1 up to and including day 7 were in the first trial 0, 22 (2), 4 (1), 0, 0 and 8 (1) for colitis-negative control, colitis-positive control, PB6 3 x 1.5 10⁸ CFU/Kg/day, PB6 3 x 1.5 10⁹ CFU/Kg/day, mesalazine 250 mg/Kg/day and infliximab 3 mg/Kg single dose respectively; and in the second trial 0, 42 (4), 10 (1), 34 (3), 12 (1) and 24 (2) for colitis-negative control, colitis-positive control, PB6 3 x 1.5 10⁸ CFU/Kg/day, *S. boulardii* 3 x 1.5 10⁸ CFU/Kg/day, mesalazine 250 mg/Kg/day and infliximab 3 mg/Kg single dose respectively. The colitis-positive control groups showed substantial body weight loss accompanying the colitis. In trial 1 this loss was higher than in trial 2, what might be related to a difference in age and growth rate at the moment of induction of colitis. The lower body weight in general at the start and the higher body weight gain in terms of percentage over the trial period of the colitis-negative control group in trial 1 probably indicate that these animals were on average somewhat younger than those in trial 2. While common treatments in trial 1 all significantly suppressed the negative effect of colitis on body weight gain, in trial 2 this was only the case with PB6 treatment (Table 11).

**Table 11. Average body weight (g) and average body weight gain (%) with their standard deviation.**

| Av. Body weight (g) | | | | Av. Body weight gain (%) |
|---|---|---|---|---|
| Trial 1 | Day 1 | Day 4 | Day 7 | Day 1 to 7 |
| Colitis-negative control | 178 ± 17 | 191 ± 13 * | 203 ± 13 * | 14.3 ± 4.5 * |
| Colitis-positive control | 182 ± 15 | 167 ± 16 | 159 ± 18 | -13.0 ± 4.5 |
| PB6 1.5 x 10⁸ CFU/Kg | 180 ± 13 | 186 ± 13 | 195 ± 10 * | 8.8 ± 8.0 * |
| PB6 1.5 x 10⁹ CFU/Kg | 180 ± 16 | 187 ± 15 | 199 ± 17* | 10.3 ± 1.1* |
| Mesalazine | 181 ± 11 | 187 ± 10 | 196 ± 10* | 8.8 ± 1.4* |
| Infliximab | 180 ± 14 | 183 ± 7 | 186 ± 6 * | 1.3 ± 5.4* |

| Trial 2 | Day 1 | Day 4 | Day 7 | Day 1 to 7 |
|---|---|---|---|---|
| Colitis-negative control | 207 ± 20 | 210 ± 19 | 214 ± 17 | 3.5 ± 1.8 * |
| Colitis-positive control | 208 ± 21 | 196 ± 21 | 193 ± 19 | -7.5 ± 3.2 |
| PB6 1.5 x 10⁸ CFU/Kg | 206 ± 23 | 206 ± 23 | 212 ± 26 | 2.9 ± 3.1 * |
| *S. boulardii* | 207 ± 17 | 196 ± 16 | 195 ± 24 | -5.7 ± 5.6 |
| Mesalazine | 207 ± 12 | 199 ± 18 | 201 ± 20 | -3.3 ± 5.5 |
| Infliximab | 208 ± 15 | 197 ± 14 | 197 ± 13 | -5.2 ± 3.4 |

| | | | | |
|---|---|---|---|---|
| *Significantly different from the positive control group (Dunnett, P<0.05). | | | | |

The greater impact of colitis on the animals in trial 1 probably left more room for improvement by any of the treatments. PB6 and mesalazine always resulted in a colon segment weight and a gross morphology score for the colon wall that could be clearly distincted from those of the colitis-positive control group (Table 12).

**Table 12. Average gross morphology score for the colon wall and average wet weight of a 5 cm colon segment.**

| | Gross morphology score | Wet weight (g) |
|---|---|---|
| Trial 1 | | |
| IBD-negative control | 0.2 ± 0.4 * | 0.378 ± 0.047 * |
| IBD-positive control | 3.8 ± 0.8 | 1.406 ± 0.209 |
| PB6 1.5 x 10⁸ CFU/Kg (post-induction) | 1.0 ± 0.0 * | 0.443 ± 0.063 * |
| PB6 1.5 x 10⁹ CFU/Kg (post-induction) | 0.6 ± 0.5 * | 0.513 ± 0.317 * |
| Mesalazine | 0.8 ± 0.4 * | 0.435 ± 0.052 |
| Infliximab | 3.0 ± 1.4 | 1.055 ± 0.490 |

| Trial 2 | | |
|---|---|---|
| IBD-negative control | 0.0 ± 0.0 * | 0.274 ± 0.049 * |
| IBD-positive control | 3.4 ± 1.1 | 0.832 ± 0.216 |
| PB6 1.5 x 10⁸ CFU/Kg | 0.6 ± 0.5 * | 0.280 ± 0.039 * |
| *S. boulardii* | 2.8 ± 1.3 | 0.735 ± 0.221 |
| Mesalazine | 1.2 ± 0.8 * | 0.445 ± 0.145 * |
| Infliximab | 2.0 ± 0.7 | 0.657 ± 0.076 |

| | | |
|---|---|---|
| *Significantly different from the positive control group (Dunnett, P<0.05). | | |

The health status of the colon wall in rats with TNBS induced colitis treated with PB6 and Mesacol was macroscopically the same as that in colitis-free rats. For an unknown reason mesalazine treatment was somewhat less effective in trial 2, resulting in some ulcerations. Visual examinations of the longitudinally opened colon segments clearly show the ulcerations and areas of necrotic tissue present in the positive control, the *S. boulardii,* and the infliximab treatment groups and the absence thereof in the PB6 groups and the trial 1 mesalazine group. In trial 1 one of the rats treated with infliximab had a gross morphology score of 1 and a colon segment weight of 0.402 g. These data suggest that in this particular animal the infliximab treatment was successfull or that the induction of colitis was not. At the end of the treatment period the average body weight gain and colon segment weight data of rats treated with infliximab were intermediary to those of the colitis-negative and the colitis-positive control groups in both trials. This probably indicates that there was some effect of infliximab, although not statistically significant in these trials. In a similar trial in rats where infliximab was administered at the same dose of 3 mg/Kg i.v. 2 days before induction of colitis with TNBS, it was shown to have some effect on colon gross morphology but not to reduce the level of edema significantly below that of the colitis-positive control group.¹⁹ In the colon segments of rats treated with PB6 there were no ulcerations. Only hyperaemia was observed in the majority of these segments. PB6 clearly attenuates inflammation in the rat colon wall as induced by intrarectal administration of TNBS. The efficacy of a 7 days post-induction treatment with 3 x 1.5 10⁹ CFU/Kg/day observed in a previous trial was confirmed in trial 1.²⁰ While a 7 days post-induction treatment with PB6 3 x 8 10⁷ CFU/Kg/day from the same previous trial was concluded to be ineffective, an increase of the CFU/Kg/day to 3 x 1.5 10⁸ proved in both current trials to be sufficient to reduce inflammation to an extent that the data of this treatment could no longer be statistically discerned from those of the colitis-negative control group.

### Conclusion

This study was designed and conducted to confirm and document the efficacy of *Bacillus* 'PB6' against 2,4,6 - trinitrobenzenesulfonic acid (TNBS)-induced colitis in rats observed in a previous trial as well as to compare its efficacy with that of *S. boulardii* (probiotic), mesalazine and infliximab (standard drugs). This rat model is well established, reliable and widely used to examine the efficiency of drugs aimed at treating IBD. Without any treatment after induction of colitis several rats showed mild signs of diarrhea and on average kept losing weight throughout the remaining trial period. The gross morphology of the intestinal wall of their colon was characterized by inflammation, ulceration and even necrosis. Treatment with PB6 3 times 1.5 10⁸ CFU/Kg/day or 3 times 1.5 10⁹ CFU/Kg/day for 7 days resulted in a colon wall health status that was statistically identical to that of the negative control group and that of the group treated with the standard drug mesalazine, an anti-inflammatory.

### REFERENCES

¹⁰ Sharma, A.K., Holer, F.E. Clostridium difficile diarrhea after use of tacrolimus following renal transplantation. Clin Infect Dis. 1998;27:1540-1541.
¹¹ Kelly, C.P. LaMont, J.T. Clostridium difficile infection. Annu Rev 1998;49:375-390
²⁰ Samore, M.H. et al Clinical and molecular epidemiology of sporadic and clustered cases of nosocomial Clostridium difficile diarrhea. Am J Med. 1996;100:32-40
²⁵ Kelly, C.P. et al. Clostridium difficile colitis. N Engl J Med 1994;330(4):257-262
²⁶ Fekety, R. and Shah, A.B. Diagnosis and treatment of Clostridium difficile colitis. JAMA 1993;269(1):71-75
²⁷ Pothoulakis, C. and LaMont, J.T. Clostridium difficile colitis and diarrhea. Gastroenterol Clin North Am 1993;22(3):623-637
²⁸ Fekety, R. et al. Recurrent Clostridium difficile diarrhea: characteristics of and risk factors for patients enrolled in a prospective, randomized, double-blinded trial. Clin Infect Dis 1997;24(3):324-333
³¹ Poduval, R.D. et al.Clostridium difficile and vancomycin-resistant enteroccoccus: the new nosocomial alliance. Am J Gastroenterol 2000;95:3513-3515
Paul A.A. C. Inulin and Oligofructose: Safe Intakes and Legal Status. Journal of Nutrition (1999) 129, 1412S-1417S.
⁴³ Gibson G.R., Probert H.M., Loo J.V., Rastall R.A., Roberfroid M.B. Dietary modulation of the human colonic microbacteria: updating the concept of probiotics. Nutr. Res. Rev. (2004) 17,259-275
⁴⁴ Gibson G.R., Roberfroid M.B. Dietary modulation of the human colonic microbiota-introducing the concept ofprobiotic. J. Nutr. (1995) 125, 1401-1412
⁴⁵ Roberfroid M. Dietary Fiber, Inulin, and Oligofructose: a Review Comparing their Physiological Effects. Crit. Rev. Food Sci. Nutri. (1993) 33, 103-148
⁴⁶ Van Loo J., Coussement P., De Leenheer L., Hoebregs H., Smits G. On the presence of inulin and oligofructose as natural ingredients in the Western diet. Crit. Rev. Food Sci. Nutr. (1995) 35, 525-552
⁴⁷ Ezaki, T., Hashimoto, Y. and Yabuuchi, E. Fluorometric deoxyribonucleic acid-deoxyribonucleic acid hybridization in microdilution wells as an alternative to membrane filter hybridization in which radioisotopes are used to determine genetic relatedness among bacterial strains. Int. J. Syst. Bacteriol. 1989; 39: 224-229
⁴⁸ Goris, J., Suzuki, K., De Vos, P., Nakase, T. and Kersters, K. Evaluation of a microplate DNA-DNA hybridization method compared with the initial renaturation method. Can J. Microbiol. 1998; 44: 1148-1153
⁴⁹ Wayne et al. Int. J. Syst. Bacteriol. 1987; 37: 463-464
⁵⁸ Kim, K. et al. Suppression of inflammatory responses by surfactin, a selective inhibitor of platelet cytosolic phospholipase A2. Biochem. Pharmacol. 1998; 55: 975 - 985.

## Claims

1. Use of a *Bacillus subtilis* bacteria of the strain identified as ATCC strain PTA-6737 in the manufacture of a medicament for the prophylaxis or treatment of inflammatory bowel disease.

2. Use according to claim 1, wherein the *Bacillus* bacteria is combined with inulin or a probiotic.

3. A *Bacillus subtilis* bacteria of the strain identified as ATCC strain PTA-6737 for use in the treatment or prophylaxis of inflammatory bowel disease.

4. A *Bacillus subtilis* bacteria according to claim 3 in combination with inulin or a probiotic for use according to claim 3.

5. Use of an isolated bacterial strain having the 16S rRNA sequence of SEQ ID NO. 1 in the manufacture of a medicament for the prophylaxis or treatment of inflammatory bowel disease.

6. An isolated bacterial strain having the 16S rRNA sequence of SEQ ID NO. 1 for use in the treatment or prophylaxis of inflammatory bowel disease.

## Patentansprüche

1. Verwendung von *Bacillus subtilis*-Bakterien des Stamms, der als ATCC-Stamm PTA-6737 identifiziert wurde, zur Herstellung eines Medikaments zur Prophylaxe oder Behandlung von chronisch-entzündlichen Darmerkrankungen.

2. Verwendung gemäß Anspruch 1, wobei die *Bacillus*-Bakterien mit Inulin oder einem Probiotikum kombiniert werden.

3. *Bacillus subtilis*-Bakterien des Stamms, der als ATCC-Stamm PTA-6737 identifiziert wurde, zur Verwendung in der Behandlung oder Prophylaxe von chronisch-entzündlichen Darmerkrankungen.

4. *Bacillus subtilis*-Bakterien gemäß Anspruch 3 in Kombination mit Inulin oder einem Probiotikum zur Verwendung gemäß Anspruch 3.

5. Verwendung eines isolierten Bakterienstamms, der die 16S rRNA-Sequenz der SEQ-ID-NR. 1 aufweist, zur Herstellung eines Medikaments zur Prophylaxe oder Behandlung von chronisch-entzündlichen Darmerkrankungen.

6. Isolierter Bakterienstamm, der die 16S rRNA-Sequenz der SEQ-ID-NR. 1 aufweist, zur Behandlung oder Prophylaxe von chronisch-entzündlichen Darmerkrankungen.

## Revendications

1. Utilisation d'une bactérie *Bacillus subtilis* de la souche identifiée comme souche ATCC PTA-6737 dans la fabrication d'un médicament pour la prévention ou le traitement d'une maladie intestinale inflammatoire.

2. Utilisation selon la revendication 1, dans laquelle la bactérie *Bacillus* est combinée avec de l'inuline ou un probiotique.

3. Une bactérie *Bacillus subtilis* de la souche identifiée comme souche ATCC PTA-6737 pour une utilisation dans le traitement ou la prévention d'une maladie intestinale inflammatoire.

4. Une bactérie *Bacillus subtilis* selon la revendication 3 en combinaison avec de l'inuline ou un probiotique pour une utilisation selon la revendication 3.

5. Utilisation d'une souche bactérienne isolée ayant la séquence ARNr 16S de SEQ ID NO. 1 dans la fabrication d'un médicament pour la prévention ou le traitement d'une maladie intestinale inflammatoire.

6. Une souche bactérienne isolée ayant la séquence ARNr 16S de SEQ ID NO. 1 pour une utilisation dans le traitement ou la prévention d'une maladie intestinale inflammatoire.
